(19) Europäisches Patentamt
European Patent Office
Office européen des brevets

(11) **EP 3 747 416 B1**

(12) # EUROPEAN PATENT SPECIFICATION

(45) Date of publication and mention
of the grant of the patent:
**16.07.2025 Bulletin 2025/29**

(51) International Patent Classification (IPC):
**A61F 13/511** (2006.01)   **A61F 13/514** (2006.01)
**D04H 3/16** (2006.01)

(21) Application number: **18909316.4**

(22) Date of filing: **13.11.2018**

(52) Cooperative Patent Classification (CPC):
**D04H 3/16; A61F 13/511; A61F 13/514**

(86) International application number:
**PCT/JP2018/042023**

(87) International publication number:
**WO 2019/176167 (19.09.2019 Gazette 2019/38)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.03.2018 JP 2018047118**

(43) Date of publication of application:
**09.12.2020 Bulletin 2020/50**

(73) Proprietor: **Unicharm Corporation
Shikokuchuo-shi, Ehime 799-0111 (JP)**

(72) Inventors:
• **MITSUNO, Satoshi
Kanonji-shi, Kagawa 769-1602 (JP)**

• **IKEUCHI, Norihito
Kanonji-shi, Kagawa 769-1602 (JP)**
• **KURITA, Noritomo
Kanonji-shi, Kagawa 769-1602 (JP)**

(74) Representative: **Dolleymores
9 Rickmansworth Road
Watford, Hertfordshire WD18 0JU (GB)**

(56) References cited:
EP-A1- 3 117 811        EP-A1- 3 275 413
WO-A1-2014/050544    WO-A1-2015/182441
WO-A1-2016/103961    CA-A1- 2 733 026
JP-A- 2015 091 353      JP-A- 2016 059 673
JP-A- 2017 104 197      KR-A- 20120 028 551
US-A1- 2016 287 450

## Description

[Technical Field]

The present invention relates to an absorbent article such as a disposable diaper and a sanitary napkin.

[Background Art]

An absorbent article using a fibrous non-woven fabric sheet is conventionally known.
For example, Patent Literature 1 discloses an absorbent article using a non-woven fabric in which at least a surface layer is formed of composite short fibers having a fiber diameter of 10 to 15 $\mu$m, a friction coefficient MIU is 0.25 or less, and a reflectance per unit weight is 1.2% or more.

[Patent Literature]

Patent Literature 1: Japanese Unexamined Patent Publication No. H11-293554
EP 3275413 A1, EP 3117811 A1, WO 2014/050544 A1, WO 2015/182441 A1, CA 2733026 A1, US 2016/287450 A1 and KR 2012 0028551 A relate to the field of absorbent articles and provide disclosures of fibrous non-woven fabrics.

[Summary of the Invention]

[Technical Problem]

The absorbent article according to the invention disclosed in Patent Literature 1 is formed of a non-woven fabric containing composite short fibers having a fiber diameter of 10 to 15$\mu$m, in which surface layers are heat-sealed to each other. The non-woven fabric has a friction coefficient MIU of 0.25 or less as a surface property, thereby being soft and excellent in texture.
In addition, since the reflectance of the non-woven fabric is 1.2% or more, a degree of gaps between the fibers of the non-woven fabric is relatively low, and therefore, a degree of exudation and/or omission of an adhesive or the like can be suppressed.
However, in such an absorbent article, since the non-woven fabric is formed of the composite short fibers, the number of thermally fused portions between the fibers is relatively large and a distance between the thermally fused portions is small, so that the non-woven fabric is difficult to deform and bend, and the drapability may be deteriorated.
In addition, since the degree of gaps between the fibers is relatively low, a portion serving as an air passage through which air passes is reduced, and air permeability is deteriorated.
Further, in a sheet manufacturing process, a defibration process by a card machine for defibrating the composite short fibers is required, and therefore, it can be said that the manufacturing cost is increased and the productivity is low as compared with a case of using continuous fibers.
An object of the present invention is to provide an absorbent article using a flexible non-woven fabric having a good texture and an appropriate air permeability.

[Solution to Problem]

In order to solve the above problem, the present invention provides the absorbent article of independent claim 1. The dependent claims specify preferred but optional features.

[Advantageous Effect of the Invention]

In the absorbent article according to the present invention, at least one of the inner and outer surface side sheets is formed of a flexible non-woven fabric, the flexible non-woven fabric is mainly formed of polyethylene fibers having a fiber diameter of 14 to 22 $\mu$m, and MMD/MIU*100 (%) of a variation coefficient to a friction coefficient obtained by a KES method is 2% to 6%, so that the absorbent article is excellent in flexibility to provide a soft and smooth tactile feel to a wearer of the absorbent article and/or a wearer's care taker, and a good air permeability can suppress an evaporation in the absorbent article.

[Brief Description of Drawings]

Fig. 1 is a perspective view of a disposable diaper in an example of an embodiment of an absorbent article according to the present invention.

Fig. 2 is a partially cutaway developed plan view of a disposable diaper extended in a longitudinal direction and a transverse direction to a point of maximum elongation of each elastic member (to the extent that the gathers under the contraction action of an elastic material disappear) when the disposable diaper is viewed from the inner surface side.

Fig. 3 is an exploded perspective view of a disposable diaper.

[Description of Embodiments]

The following embodiments relate to a disposable diaper 10 in one example of an embodiment of an absorbent article according to the present invention and include not only essential but also selected and preferred configurations of the invention.

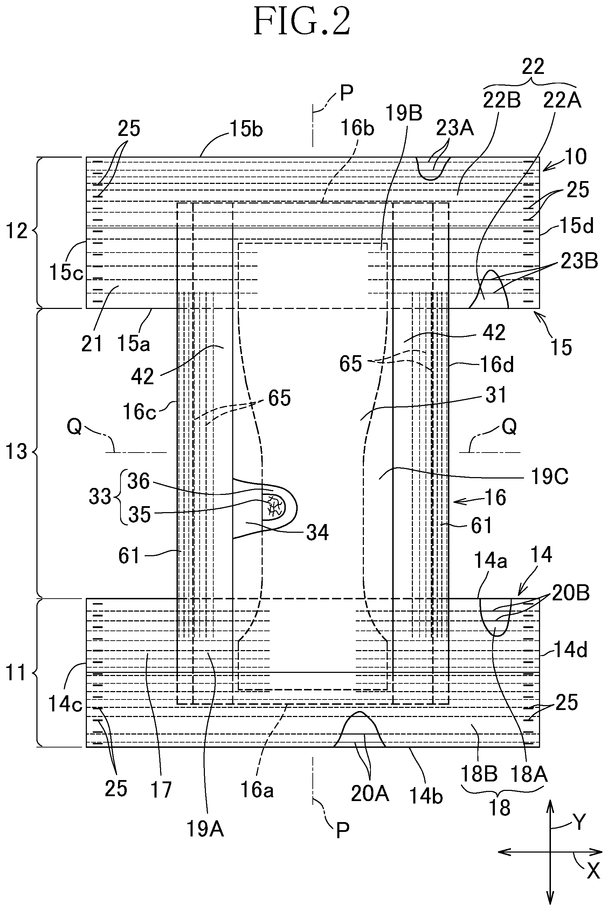

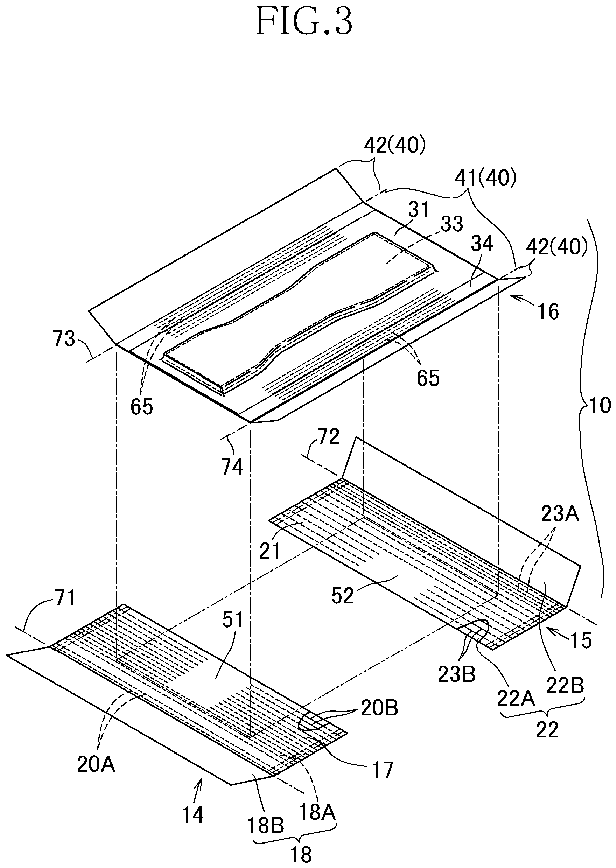

In Figs. 2 and 3, each elastic member to be described later, is in a state of being elongated to such an extent that gathers generated in a member to which each elastic member is attached appear to be substantially eliminated by natural vision due to a contraction force in a transverse direction X and a longitudinal direction Y against the contraction force of the elastic member.

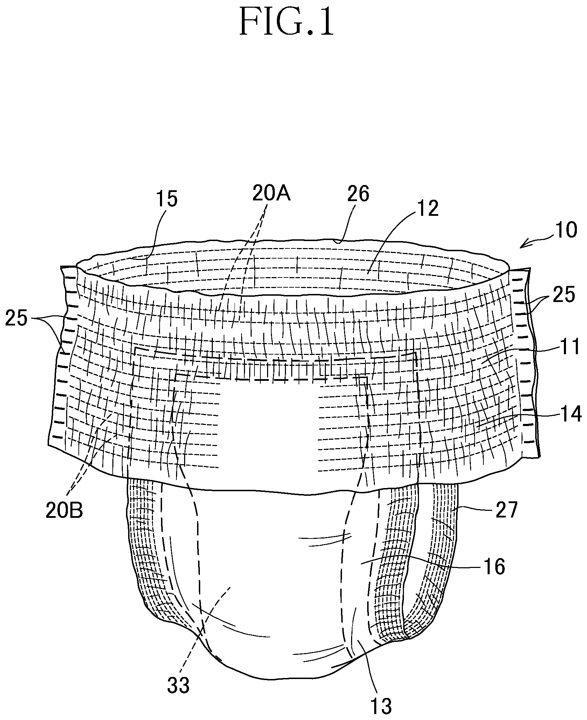

When referring to Figs. 1 to 3, the disposable diaper 10, which is an example of a disposable absorbent article according to the present invention, has the longitudinal direction Y and the transverse direction X intersecting therewith, a thickness direction Z, a skin facing surface, and a non-skin facing surface opposite thereto, a longitudinal centerline P bisecting a length dimension of the transverse direction X, a transverse centerline Q bisecting a length dimension of the longitudinal direction Y, is substantially symmetrical with respect to the longitudinal centerline P and includes a front waist region 11, a rear waist region 12, and a crotch region 13 located between the front waist region 11 and the rear waist region 12.

In this matter, the absorbent article according to the embodiment of the present invention includes, in addition to a disposable diaper and a sanitary napkin, various well-known articles including a panty liner and a urine taking pad for absorbing and holding body fluids, for example, body exudates such as menstrual blood, feces and urine or the like.

The diaper 10 includes a front waist panel 14 forming the front waist region 11, a rear waist panel 15 forming the rear waist region 12, and an absorbent panel 16 extending in the longitudinal direction Y between the front and rear waist panels 14 and 15 to form the crotch region 13.

The front waist panel 14 and the rear waist panel 15 are formed to have the same shape and size, for example.

The absorbent panel 16 includes a front end portion 19A located on the skin facing surface side of the front waist region 11, a rear end portion 19B located on the skin facing surface of the rear waist region 12, and an intermediate portion 19C located between the front and rear end portions 19A and 19B.

In the present embodiment, the front and rear end portions 19A and 19B of the absorbent panel 16 are located and fixed on the skin facing surface side of the front and rear waist regions 11 and 12, respectively, but the absorbent panel 16 may be fixed to the outer surfaces of the front and rear waist panels 14 and 15 so that the front and rear end portions 19A and 19B are located on the non-skin facing surfaces of the front and rear waist regions 11 and 12.

Both side edge parts of the front waist region 11 and both side edge parts of the rear waist region 12 are bonded to each other by a plurality of seams 25 which are spaced apart in the longitudinal direction Y, to define a waist opening 26 and a pair of leg openings 27.

The seam 25 can be formed by adhesive means using various adhesives such as a hot melt adhesive, various known bonding means using welding means such as heat sealing or sonic, or a combination thereof.

The front waist panel 14 has a horizontal rectangular shape defined by an inner edge (inner edge of the front waist region) 14a forming a part of the front leg opening, an outer edge (outer edge of the front waist region) 14b forming the front waist opening, and both side edges 14c and 14d (both side edges of the front waist region) extending in the longitudinal direction Y between the inner and outer edges 14a and 14b.

When referring to Figs. 2 and 3, the front waist panel 14 includes an impermeable inner layer sheet 17 located on the skin facing surface side and an impermeable outer layer sheet 18 located on the non-skin facing surface side.

The outer layer sheet 18 includes a main portion 18A which is bonded to the inner layer sheet 17, and a folded portion 18B extending outwardly in the longitudinal direction Y from the outer edge of the inner layer sheet 17.

The folded portion 18B is folded inward in the longitudinal direction Y along the folding line 71 extending in the transverse direction X to be fixed to the skin facing surface sides of the inner layer sheet 17 and the front end portion 19A so as to cover the front end portion 19A of the absorbent panel 16, being disposed on the skin facing surface side of the front waist panel 14.

Between the inner layer sheet 17 and the main portion 18A of the outer layer sheet 18, a plurality of strand-like or string-like front waist elastic members 20 are fixed so as to be contractible in the transverse direction X.

The rear waist panel 15 includes an inner edge (inner edge of the rear waist region) 15a forming a part of the rear leg opening, an outer edge (outer edge of the rear waist region) 15b forming the rear waist opening, and both side edges 15c and 15d (both side edges of the rear waist region) extending in the longitudinal direction Y between the inner and outer edges 15a and 15b.

The rear waist panel 15 includes an impermeable inner layer sheet 21 located on the skin facing surface side and an impermeable outer layer sheet 22 located on the non-skin facing surface side.

The outer layer sheet 22 includes a main portion 22A which is bonded to the inner layer sheet 21, and a folded portion 22B extending outwardly in the longitudinal direction Y from the outer edge of the inner layer sheet 21.

The folded portion 22B is folded inward in the longitudinal direction Y along the folding line 72 extending in the transverse direction X to be fixed to the skin facing surface side of the inner layer sheet 21 and the rear end portion 19B so as to cover the rear end portion 19B of the absorbent panel 16, being disposed on the skin facing surface side of the rear waist panel 15.

Between the inner layer sheet 21 and the main portion 22A of the outer layer sheet 22, a plurality of strand-like or string-like rear waist elastic members 23 are fixed so as to be contractible in the transverse direction X.

The front waist elastic member 20 includes a plurality of front upper waist elastic members 20A extending in the transverse direction X along the outer edge 14b, and a front lower waist elastic member 20B located between the front upper waist elastic member 20A and the inner edge 14a.

The rear waist elastic member 23 includes a plurality of rows of rear upper waist elastic members 23A extending in the transverse direction X along the outer edge 15b, and a rear lower waist elastic member 23B located between the rear upper waist elastic member 23A and the inner edge 15a.

The front lower waist elastic member 20B is located so as to overlap with both sides of the front end portion 19A of the absorbent panel 16 in plan view of the diaper 10, and the rear lower waist elastic member 23B is located so as to overlap with both sides of the rear end portion 19B of the absorbent panel 16 in plan view.

The front and rear lower waist elastic members 20B and 23B are made inelastic by being cut and removed at the central portions of the front and rear end portions 19A and 19B of the absorbent panel 16, respectively, and inelastic regions 51 and 52 in which the contraction force of the front and rear lower waist elastic members 20B and 23B does not act are defined at the central portions.

In the front and rear waist elastic members 20 and 23, the front and rear, upper and lower waist elastic members 20A, 20B, 23A and 23B are a plurality of thread-like, string-like and/or strand-like elastic materials extending in the transverse direction X at intervals from each other in the longitudinal direction Y.

The plurality of elastic members 20A, 20B, 23A and 23B are attached in a state of being elongated, for example, 1.5 to 3.0 times and preferably 2.2 to 2.7 times, between the inner and outer layer sheets 17, 18, 21 and 22.

In this matter, an elongation ratio 1.0 indicates an elongation degree when the length (natural length) of each elastic member in the non-elongated state is set to be 1, and for example, the elongation ratio 1.5 means that the elongation is increased by 0.5 from the length of each elastic member in the non-elongated state.

As the elastic material, in addition to natural rubber, various known synthetic rubbers such as styrene rubber, urethane rubber, ester rubber, polyurethane, and polyethylene can be used without limitation.

The fineness of each elastic member is not particularly limited, but is 200 to 1100 dtex and preferably 300 to 1000 dtex.

In a case where the fineness is 200 dtex or less, there is a concern that the required stretchability cannot be exhibited in a state in which the shape and size of the gathers are controlled by the pitch between the elastic members 20 and 23, respectively.

In a case where the fineness of each elastic member is 1100 dtex or more, each elastic member is easy to be seen through from the outside via the outer layer sheets 18 and 22 located on the non-skin facing surface side of the diaper 10 and the skin may be seen through the outer layer sheets 18 and 22.

A separation dimension (pitch) of each of the elastic members 20 and 23 is not particularly limited because it can be appropriately adjusted to control the cross-sectional shape and size including a length of the gathers formed on the inner and outer layer sheets 17, 18, 21, and 22, but is, for example, 2.0 to 12.0 mm, preferably 4.0 to 10.0 mm in order to suppress the gathers from biting into the wearer's skin.

In this specification, the pitch means the distance between the centers of the elastic members adjacent to each other in the longitudinal direction Y.

In the present embodiment, the inner and outer layer sheets 17, 18, 21, and 22 are bonded to each other via an adhesive that is continuously or partially applied to the entire circumference of each elastic member interposed therebetween.

Therefore, it can be also said that the pitch between each elastic member is, the separation dimension (pitch) of the bonding lines for bonding the inner and outer layer sheets 17, 18, 21, and 22 to each other.

Since the inner and outer layer sheets 17, 18, 21, and 22 are fixed to each other only by the adhesive applied to the entire circumference of each elastic member 20 and 23, an elastic sheet layer formed of the inner layer sheets 17, 18, 21, and 22 and the front and rear waist elastic members 20 and 23 is relatively flexible in combination with the fact that any one of the inner and outer layer sheets 17, 18, 21, and 22 is formed of the flexible non-woven fabric to be described later.

Although not shown in the drawings, sheets having elastic stretchability may be arranged in addition to or instead of the front and rear upper waist elastic members 20A and 23A on the waist opening side of the front and rear waist panels 14 and 15.

In such a case, it fits more gently to the wearer's skin than a case of having a thread-like elastic material.

The absorbent panel 16 has a vertically long rectangular shape defined by front and rear edges 16a and 16b and both side edges 16c and 16d, and front and rear end portions 19A and 19B are bonded to each other via front and rear bonding regions (not shown) formed by applying a hot melt adhesive to the skin facing surfaces of the front and rear waist panels 14 and 15.

As the application pattern of the hot melt adhesive in the front and rear bonding regions, for example, in addition to a plurality of lines extending in the longitudinal direction Y, various known shapes such as a Ω shape, a spiral shape, and a wave shape can be adopted.

When referring to Figs. 2 and 3, the absorbent panel 16 includes a hydrophilic/permeable inner sheet (body side liner) 31 located on the skin facing surface side, an absorbent body 33, a hydrophobic covering sheet 40 located on the non-skin facing surface side of the absorbent body 33, and a leakage barrier film 34 located between the absorbent body 33 and the covering sheet 40 and formed of a hydrophobic or impermeable plastic film having enough size to cover at least the bottom surface of the absorbent body 33.

The absorbent body 33 includes a liquid-absorbing/liquid absorbent core 35 and a core wrap sheet 36 covering the entire liquid absorbent core 35.

The liquid-absorbing core 35 is in the form of a semirigid panel shaped into a required shape, and includes front and rear edges extending in the transverse direction X, and both side edges extending between the front and rear edges in a convex curve toward the longitudinal centerline P (inward in the transverse direction X).

In addition, the liquid-absorbing core 35 is formed of a mixture of fluff wood pulp and superabsorbent polymer particles (SAP).

The covering sheet 40 includes a main portion 41 in which the leakage barrier film 34 is disposed, and both side portions 42 located outside in the transverse direction X from folding lines 73 and 74 extending in the longitudinal direction Y along both side edges of the leakage barrier film 34.

The both side portions 42 are folded to the longitudinal centerline P side along the folding lines 73 and 74 to be overlapped with the main portion 41.

Between the both side portions 42 and the leakage barrier film 34, a plurality of string-like or strand-like leg elastic members 65 extending in the longitudinal direction Y are attached so as to be contractible in an elongated state.

For the leg elastic member 65, it is possible to use, for example, an elastic material which has a fineness of 470 to 740 dtex and is elongated 1.7 to 2.3 times from a contracted or relaxed state to be fixed.

A leg elastic region 61 in which the leg elastic member 65 is disposed is formed at the leg opening edge part of the diaper 10, and the leg opening edge part fits the wearer's thigh by the contraction action of the leg elastic region 61, and thereby transverse leakage of body exudates can be effectively suppressed.

<Flexible non-woven fabric>

In the diaper 10, at least one of the inner surface side sheet located on the skin facing surface side of the absorbent body 33 and the outer surface side sheet located on the non-skin facing surface side of the absorbent body 33 is formed of a flexible non-woven fabric.

Here, the "inner surface side sheet" means a sheet located on the skin facing surface side of the absorbent body 33, and includes a body side liner 31 in the present embodiment.

In addition, although not shown in the drawings, as an option, in a case where a second sheet having a high cushioning property is disposed between the absorbent body 33 and the body side liner 31, the second sheet also corresponds to the inner surface side sheet.

The "outer surface side sheet" is a sheet located on the non-skin facing surface side of the absorbent body 33, and in the present embodiment, includes the inner and outer layer sheets 17, 18, 21, and 22 forming the front and rear waist panels 14 and 15 in addition to the covering sheet 40 of the absorbent panel 16.

In a case where a flexible non-woven fabric is used as the hydrophilic body side liner 31, it is necessary to mix hydrophilic fibers or to separately apply a hydrophilic oil agent to the hydrophobic fibers.

Further, in the diaper 10, a plurality of the front and rear waist elastic members 20 and 23 extending in the transverse direction X are arranged on the inner and outer layer sheets 17, 18, 21, and 22 forming the front and rear waist panels 14 and 15, and in order to facilitate manufacturing, the transverse direction X is a machine direction MD (flow direction of the line at the time of manufacturing non-woven fabric) of the sheet as a substrate of the inner and outer layer sheets 17, 18, 21, and 22, and the longitudinal direction Y is a direction CD that intersects therewith.

On the other hand, in the absorbent panel 16, a plurality of the leg elastic members 65 extending in the longitudinal direction Y are arranged, and in order to facilitate manufacturing, the longitudinal direction Y is the machine direction MD of the sheet as a substrate of each of the sheets 31, 34, and 40 forming each of the leg elastic members, and the transverse direction X is the cross direction CD.

The flexible non-woven fabric is a fiber non-woven fabric containing a thermoplastic synthetic resin, and as the flexible non-woven fabric, a spun-melt fiber non-woven fabric, in particular, a spunbond fiber non-woven fabric or a SMS (spunbond-meltblown-spunbond) fiber non-woven fabric is suitably used.

In addition, as the continuous fibers forming these non-woven fabrics, polyolefin thermoplastic fibers containing polypropylene (PP) and polyethylene (PE), which are various known synthetic fibers, are suitably used.

In the present embodiment, a spunbond fiber non-woven fabric mainly containing polyethylene fibers is used as the flexible non-woven fabric.

Since the flexible non-woven fabric is formed of a spun-melt non-woven fabric made of continuous fibers, unlike an air-laid non-woven fabric or the like containing short fibers, free ends of the fibers do not come into direct contact with the skin of the wearer.

In particular, when the fingertip of the wearer touches the fabric, the free end of the fiber does not hit the fingerprint, and the fabric is formed of continuous fibers and highly free, and thus the fabric can be touched so as to stick to the fingerprint of the finger, thereby providing soft and good texture.

In addition, unlike the case of using the composite fibers or the like, a fiber defibration step is not required, thereby the manufacturing process is less and the manufacturing cost can be suppressed.

By using a spunbond fiber non-woven fabric or an SMS fiber non-woven fabric formed of the continuous fibers, the flexible non-woven fabric can provide a smooth and soft tactile feel without fluffing on the sheet surface as compared with other synthetic fibers formed of the short fibers.

In addition, the spunbond fiber non-woven fabric is easier to control the fiber orientation than other fiber non-woven fabrics.

Therefore, for example, in a case where a staple (short fiber) fiber non-woven fabric is used as the inner and outer layer sheets 17, 18, 21, and 22 with the front and rear waist elastic members 20 and 23 interposed therebetween, it is excellent in the flexibility and elongation but inferior in the sheet tensile strength, while it is possible to obtain a well-balanced composite elastic sheet having a desired sheet tensile strength and elongation by using the spunbond fiber non-woven fabric.

Here, the "smooth and soft tactile feel" of the flexible non-woven fabric includes the tactile feel by the wearer who wears the diaper 10, as well as the tactile feel by the wearer's caretaker such as a mother who assists the wearer in wearing the same when the wearer is an infant or the like.

In particular, when assisting wearing, the wearer's caretaker determines the flexibility and good texture of the diaper 10 based on the tactile feel when a part of the inner and outer surface side sheets of the diaper 10 touches the fingertip.

Specifically, when the wearer's caretaker touches the flexible non-woven fabric forming the inner and outer surface side sheets of the diaper 10, a tactile feel such that the fibers stick to the fingerprint of the finger is provided so as to have the caretaker recognize that the diaper 10 is excellent in flexibility and texture.

The flexible non-woven fabric is a fiber non-woven fabric mainly containing polyethylene fibers, and the content of the polyethylene fibers is 100% by mass with respect to the entire sheet.

In addition, since the polyethylene fiber has a relatively large fiber diameter, a fiber gap is relatively large and the air permeability is excellent.

Therefore, by using the flexible non-woven fabric as the inner and outer surface side sheets of the diaper 10, the sheets gently fit the skin of the wearer and the evaporation inside the diaper 10 can be suppressed.

In a case where the flexible non-woven fabric is formed by a spunbonded non-woven fabric using continuous polyethylene fibers, the continuous fibers have a plurality of fused portions which are thermally fused to each other by a heat treatment using a calender embossing method.

In the fused portion, each of the constituent fibers is thermally fused and does not maintain the fiber shape, and a part thereof is formed into a film, so that the rigidity is higher than that of the non-fused portion of each of the constituent fibers.

The total area ratio of the fused portion to the area of the flexible non-woven fabric is related to the texture and sheet strength of the flexible non-woven fabric, but from this viewpoint, it is preferable that the total area ratio of the fused portion is 5% to 25%.

In a case where the total area ratio is 5% or less, the sheet strength may be low and a part may be broken during wearing of the absorbent wearing article, whereas in a case where the total area ratio is 25% or more, the flexible non-woven fabric may be hardened as a whole and the texture may be deteriorated.

The continuous fiber non-woven fabric made of thermoplastic resins, which are commonly used in this type of field, is excellent in smoothness without fluffing because of the absence of free ends of fibers, but is generally hard in texture and lacks flexibility.

Although some improvements can be made by appropriately changing the design such as heat treatment conditions during manufacturing, it is not possible to obtain a non-woven fabric having sufficient softness, smoothness, and air permeability.

The applicant, as the inner and outer surface side sheets of the diaper 10, uses the flexible non-woven fabric which is formed mainly by polyethylene fibers and is formed of the spun-melt non-woven fabric having a fiber diameter in a predetermined range and a variation coefficient of a friction coefficient in accordance with the KES method, and found that the sheets are excellent in smoothness without fluffing and provide the wearer and the wearer's caretaker with smooth and soft tactile feel.

The superiority of using the flexible non-woven fabric as the inner and outer surface side sheets of the diaper 10 can be exemplified by "smoothness", "soft tactile feel", and "good air permeability", and the superiority can be confirmed by surface property, flexural property, fiber density, fiber diameter, specific volume, compression property, and the like in the flexible non-woven fabric.

The mass of the flexible non-woven fabric is, for example, 10 to 30 $g/m^2$ and preferably 12 to 25 $g/m^2$, and the thickness dimension under a slight load (0.49 hPa) is 0.10 to 0.60 mm.

In a case where the mass of the flexible non-woven fabric is less than 10 $g/m^2$, it is difficult to obtain sufficient tensile strength although the flexibility is excellent.

In a case where the mass of the flexible non-woven fabric exceeds 30 $g/m^2$, a relatively high tensile strength can be obtained, but sufficient flexibility cannot be obtained, and the air permeability may be lowered.

The apparent density of the flexible non-woven fabric under a load of 0.49 hPa ($\fallingdotseq$ 0.5 $gf/cm^2$) is 0.02 to 0.1 $g/cm^3$ and preferably 0.04 to 0.08 $g/cm^3$, and the apparent density under a load of 49.03 hPa is 0.1 to 0.2 $g/cm^3$ and preferably 0.12 to 0.18 $g/cm^3$.

In addition, the specific volume of the flexible non-woven fabric under a load of 0.49 hPa is 10 to 50 $cm^3/g$ and preferably 12.0 to 18.0 $cm^3/g$, and the specific volume under a load of 49.03 hPa is 5.0 to 10 $cm^3/g$ and preferably 4.0 to 8.0 $cm^3/g$.

In a case where the apparent density of the flexible non-woven fabric under a load of 0.49 hPa is less than 0.02 $g/cm^3$, the fused portions in the flexible non-woven fabric are reduced, and thereby the flexible non-woven fabric may easily fluff.

On the other hand, in a case where the apparent density under a load of 0.49 hPa exceeds 0.1 $g/cm^3$, the rigidity of the flexible non-woven fabric is relatively high, and particularly in a case where the flexible non-woven fabric is used as an inner surface side sheet in a portion being in contact with a weak skin portion such as a groin of the wearer, the wearer may have hard tactile feel and the air permeability may be lowered.

When the specific volume under the 49.03hPa ($\fallingdotseq$ 50$gf/cm^2$) load of the flexible non-woven fabric falls within a predetermined range and the fiber is compressed by being pushed by the fingertip, the density is increased because the fiber is easily collapsed and has the relatively low repellency, the area of contact between the fingertip and the fiber is relatively large, the fiber is easily stuck around the fingertip, and a soft tactile feel can be provided.

The mass of the flexible non-woven fabric was measured in accordance with JISL 1096 method.

The apparent density was determined from an average value (N = 3) calculated by the mass and thickness dimensions obtained by these measurements.

The specific volume of the flexible non-woven fabric was calculated by dividing the mass of the non-woven fabric by the thickness.

The average fiber diameter of the constituent fibers (mainly polyethylene fibers) of the flexible non-woven fabric is 14 to 22 $\mu$m.

Since the fiber diameter of the flexible non-woven fabric is relatively large, it can be said that the number of fibers per unit area is relatively small and the fiber density is suppressed to be low, thereby a large number of fiber gaps are formed to have good air permeability.

<Flexural property >

A flexural rigidity value B in accordance with a KES method for flexible non-woven fabrics is 0.003 x $10^{-4}$ to 0.01 x $10^{-4}$ ($N \cdot m^2/m$).

In a case where the flexural rigidity value B of the flexible non-woven fabric is less than 0.003 x $10^{-4}$($N \cdot m^2/m$), the tensile strength of the flexible non-woven fabric is low, and a part may be broken during wearing of the diaper 10.

In a case where it exceeds 0.01 x $10^{-4}$($N \cdot m^2/m$), the sheet rigidity is too high to adapt the sheet to the body shape of the wearer, and thereby a fit to the body may be deteriorated.

Further, by the fact that the flexural rigidity value B is 0.01 x $10^{-4}$($N \cdot m^2/m$) or less, the fiber is easily flexural, the contact distance between the fiber and the fingertip in contact with the fiber increases, and the friction coefficient MIU increases.

As a result, MMD/MIU*100 (%) of a variation coefficient to the friction coefficient is 2% to 6%, and thereby the variation is less and the sheet is smoother.

That is, it can be said that smooth tactile feel can be achieved by increasing the number of fibers sticking to the fingerprint of the fingertip.

<Compression property>

A compression workload WC of the flexible non-woven fabric in accordance with the KES method is 0.14 to 0.2 N·m$^2$/m, and a compression recovery rate RC is 15% to 50%.

Since both the compression workload WC and the compression recovery rate RC are relatively low values, it can be said that the flexible non-woven fabric is easily compressed by an external force and the shape restorability after compression is low.

Therefore, for example, when the inner layer sheets 17 and 21 are formed of a flexible non-woven fabric and a plurality of small gathers are formed on the surfaces of the inner layer sheets 17 and 21 by the contraction action of the front and rear waist elastic members 20 and 23, the gathers are easily compressed when in contact with the wearer's skin, the shape restoring property after compression is low, a repulsion force against the skin is low, and therefore gathering marks can be suppressed from being left on the skin in combination with a dispersion of the contact pressure.

Also, the flexible non-woven fabric is mainly formed of flexible polyethylene fibers and has the compression recovery rate RC of less than 50%, and thereby the fibers collapse and the fiber density increases when the fingertip of the wearer's caretaker touches the surface of the sheet. The number of fibers in contact with the finger increases and soft tactile feel can be provided.

<Surface property>

The flexible non-woven fabric is a fiber web formed by thermally fusing the continuous fibers mainly formed of polyethylene fibers to each other, and since the free ends of the fibers are not present on the surface, it can be said that the surface of the sheet has good smoothness with less fluffing.

In addition, since the polyethylene fibers are more flexible than polypropylene fibers, the surface is more flexible and can deform under a slight load to softly follow and fit the movement of the wearer's body as it moves.

The surface of the flexible non-woven fabric has a friction coefficient MIU of 0.15 to 0.40, preferably 0.25 to 0.35, and an average deviation MMD of the friction coefficient of 0.008 to 0.01.

Since the friction coefficient MIU exceeds 0.15, the frictional resistance is relatively high, and when the wearer touches the surface of the sheet, it is possible to provide the wearer with the smooth tactile feel sticking to the skin.

Also, MMD/MIU*100 (%) of a variation coefficient to the friction coefficient obtained is 2.0% to 6.0%.

As described above, the compression recovery rate RC of less than 50% causes the fibers to collapse and the fiber density to increase even under a slight load when the wearer's fingertip touches, thereby the amount of fibers in contact with the fingertip increases and the friction coefficient MIU increases.

As a result, since the MMD/MIU*100 (%) of the variation coefficient to the friction coefficient falls within a relatively small range of predetermined value, the variation of the friction coefficient is less, and as a whole, it has good smoothness, and relatively many fibers come into contact with and stick to the fingerprints of the wearer's caretaker's fingertips, thereby providing smooth and soft tactile feel.

The range of predetermined values of the flexural property and the surface property in the flexible non-woven fabric is satisfied in both the machine direction MD and the cross direction CD at the time of manufacturing the non-woven fabric.

In particular, with respect to the surface property of the inner layer sheets 17 and 21 of the front and rear waist panels 14 and 15 in contact with the skin around the waist of the wearer, it is preferable that the frictional resistance of the non-woven fabric in the machine direction MD (transverse direction X of the diaper as the product) falls within a range of predetermined value.

In such a case, since the variation coefficient to the friction coefficient in the transverse direction X is relatively small, when an infant having a body shape with an expanding belly wears the diaper, a wearing operation can be performed smoothly without the inner layer sheets 17 and 21 being caught by the body when the front and rear waist panels 14 and 15 are elongated in a waist-around direction Further, when the variation coefficient of the friction coefficient in the cross direction CD (longitudinal direction Y of the diaper as a product) of the non-woven fabric forming the inner layer sheets 17 and 21 is relatively small, in pulling up the diaper 10 having passed through both legs of the wearer such as an infant, it can be smoothly worn without causing any irritations even if the inner surface side sheet made of the flexible non-woven fabric is in sliding contact with the skin of the infant which is softer than that of the adult.

On the other hand, when the fingertip of the wearer's caretaker touches an outer surface side sheet formed of a flexible non-woven fabric and located on the non-skin facing surface side of the waist region of the diaper, the fingertip moves on the sheet surface in a random plane direction regardless of the longitudinal direction Y and the transverse direction X, and therefore, the flexural property and the surface property in either of the two directions X and Y may fall within a range of predetermined value.

As described above, in the flexible non-woven fabric according to the present embodiment, since the fiber diameter, the fiber density, and the specific volume fall within a predetermined range, the number of fibers is relatively small, and the air permeability is excellent.

In addition, even in a case where the fiber diameter of the constituent fibers of the flexible non-woven fabric is relatively large, the irregularities on the surface of the sheet easily collapse by being formed mainly of the flexible polyethylene fibers, and the distribution of the friction coefficient is small at a place where the friction coefficient is high, so that it is possible to provide the wearer and the wearer's caretaker with a smooth and soft tactile feel so as to stick to the skin.

Each dynamic measurement in accordance with the KES method in this specification is explained in detail in the second edition of "Standardization and Analysis of Texture Hand Evaluation" (published July 10, 1980 by the Texture Machinery Society of Japan).

Table 1 indicates evaluation for the properties and performance to compare a plurality of flexible non-woven fabrics manufactured under various conditions with the non-woven fabrics conventionally used in this type of field.

The flexible non-woven fabrics of Examples 1 to 5 are formed of a spunbonded non-woven fabric containing 100% by mass of polyethylene fibers, which are continuous fibers, and the non-woven fabric of Comparative Example 1 is formed of a spunbonded non-woven fabric containing 100% by mass of polypropylene fibers, which are continuous fibers.

The flexible non-woven fabrics of Examples 1 to 5 and the non-woven fabrics of Comparative Examples 1 and 2 were subjected to a calendering heat treatment by a hot embossing roll in the manufacturing process.

[Table 1]

| | | Example 1 | Example 2 | Example 3 | Example 4 | Example 5 | Comparative Example 1 | Comparative Example 2 | Comparative Example 3 |
|---|---|---|---|---|---|---|---|---|---|
| Mass | $(g/m^2)$ | 20.40 | 18.98 | 20.84 | 25.12 | 15.14 | 20.37 | 15.08 | 23.52 |
| Average fiber diameter | $(\mu m)$ | 14.83 | 16.89 | 20.28 | 16.77 | 16.77 | 12.19 | 17.35 | 16.64 |
| Density | Under 0.49 hPa load $(g/cm^3)$ | 0.07 | 0.07 | 0.08 | 0.07 | 0.06 | 0.06 | 0.08 | 0.01 |
| | Under 49.03 hPa load $(g/cm^3)$ | 0.13 | 0.15 | 0.18 | 0.13 | 0.12 | 0.12 | 0.16 | 0.07 |
| Specific volume | Under 0.49 hPa load $(cm^3/g)$ | 15.10 | 13.75 | 12.28 | 14.73 | 17.24 | 17.57 | 12.20 | 71.85 |
| | Under 49.03 hPa load $(cm^3/g)$ | 7.50 | 6.74 | 5.61 | 7.44 | 8.06 | 8.54 | 6.30 | 14.46 |
| Surface property | MIU | 0.335 | 0.290 | 0.173 | 0.268 | 0.285 | 0.238 | 0.130 | 0.137 |
| | MMD | 0.0086 | 0.0104 | 0.0100 | 0.0083 | 0.0081 | 0.0078 | 0.0083 | 0.0084 |
| | MMD/MIU*100 (%) | 2.57 | 3.59 | 5.78 | 3.10 | 2.84 | 3.28 | 6.38 | 6.13 |
| Flexural property | B (N*m$^2$ / m*10^-4) | 0.0065 | 0.0060 | 0.0067 | 0.0096 | 0.0035 | 0.0202 | 0.0106 | 0.0826 |
| Compression property | TO (thickness under 0.49 hPa load) (mm) | 0.31 | 0.26 | 0.26 | 0.37 | 0.26 | 0.36 | 0.18 | 1.69 |
| | Tm (thickness under49.03 hPa load) (mm) | 0.15 | 0.13 | 0.12 | 0.19 | 0.12 | 0.17 | 0.10 | 0.34 |
| | WC (N*m / m$^2$) | 0.16 | 0.14 | 0.15 | 0.20 | 0.14 | 0.19 | 0.10 | 1.60 |
| | RC (%) | 46.54 | 47.58 | 44.64 | 45.63 | 45.42 | 62.63 | 59.91 | 59.31 |
| Air permeability | Air-permeable resistance value (KPa · sec/m) | 0.0324 | 0.0216 | 0.0236 | 0.0380 | 0.0174 | 0.0356 | 0.0192 | 0.0122 |

EP 3 747 416 B1

<Example 1>

As a flexible non-woven fabric of Example 1, a spunbonded non-woven fabric formed of polyethylene fibers having a mass of 20.40 g/m$^2$ and an average fiber diameter of 14.83 $\mu$m was used.

<Example 2>

As a flexible non-woven fabric of Example 2, a spunbonded non-woven fabric formed of polyethylene fibers having a mass of 18.98 g/m$^2$ and an average fiber diameter of 16.89 $\mu$m was used.

<Example 3>

As a flexible non-woven fabric of Example 3, a spunbonded non-woven fabric formed of polyethylene fibers having a mass of 20.84 g/m$^2$ and an average fiber diameter of 20.28 $\mu$m was used.

<Example 4>

As a flexible non-woven fabric of Example 4, a spunbonded non-woven fabric formed of polyethylene fibers having a mass of 25.12 g/m$^2$ and an average fiber diameter of 16.77 $\mu$m was used.

<Example 5>

As a flexible non-woven fabric of Example 5, a spunbonded non-woven fabric formed of polyethylene fibers having a mass of 15.14 g/m$^2$ and an average fiber diameter of 16.77 $\mu$m was used.

<Comparative Example 1>

As the non-woven fabric of Comparative Example 1, a spunbonded non-woven fabric formed of polypropylene fibers having a mass of 20.37 g/m$^2$ and an average fiber diameter of 12.19 $\mu$m was used.

<Comparative Example 2>

As non-woven fabric of Comparative Example 2, a spunbonded non-woven fabric formed of composite fibers having a mass of 15.08 g/m$^2$ and an average fiber diameter of 17.35 $\mu$m was used.
The composite fibers were the continuous fiber, and a core-sheath type composite fiber including a core portion of polypropylene and a sheath portion of polyethylene was used.

<Comparative Example 3>

As the non-woven fabric of Comparative Example 4, an air-through non-woven fabric formed of composite fibers having a mass of 23.52 g/m$^2$ and an average fiber diameter of 16.64 $\mu$m was used.
As the composite fiber, a core-sheath type composite fiber, which is a short fiber, having a fiber length of 51 mm, and including a core portion made of polyethylene terephthalate (PET) and a sheath portion made of polyethylene was used.
The following methods were used to measure the mass, fiber diameter, surface property (MIU, MMD, MMD/MIU), flexural property (B), compression property (T0, Tm, WC, RC), and air permeability (air-permeable resistance value) of each non-woven fabric.

<Method of measuring mass>

Samples (N = 10) having a size of 100 mm wide and 200 mm long were obtained from each non-woven fabric, and the mass of the samples was determined and the average value was taken as the mass of each non-woven fabric.

<Method of measuring average fiber diameter>

First, a 10 mm × 10 mm sample of each non-woven fabric was cut out and prepared and placed on top of the preparation.
Next, an appropriate amount of glycerin was added by drops to each sample so that the entire sample was immersed in glycerin, and a cover glass was placed thereon.

Next, the sheet surface of the sample was observed at a magnification of 1000 times by using a known optical microscope (for example, a VHC-100 Digital Microscope Lens VH-Z450 made of KEYENCE), and the fiber diameter of the fiber (N = 50) exposed on the sheet surface was measured, and an average value was set as an average fiber diameter.

<Method of measuring surface property>

The surface property was measured by using a KES-FB4-A-AUTO (automated surface tester) manufactured by Kato-tec Corporation., using 100 mm * 100 mm range in a given region of each non-woven fabric as a sample, and placing each non-woven fabric on a testing stand having a smooth metallic plane.

Each sample was moved horizontally at a constant speed of 0.1 cm/sec by 0 to 2 cm in the machine direction (direction of fiber orientation) at the time of manufacturing each non-woven fabric, and the average friction coefficient in a moving section was calculated as MIU and the standard deviation of the friction coefficient at that time was calculated as MMD, under the condition of an initial load of 50 gf, by using a frictional element (measuring element) with external dimensions of 5 mm × 5 mm.

Similar measurements were performed five times for each sample, and the average was used as the MIU and MMD values for each sample.

<Method of measuring thickness dimensions T0 and Tm and compression specification>

The thickness dimension of each of the non-woven fabrics was measured using a KES-FB3-AUTO-A compression tester manufactured by Kato-tec Corporation.

First, each non-woven fabric is cut out to a size of 100 mm * 100 mm to obtain a sample, and the center of the sample is gently sandwiched by disks located above and below to measure a thickness dimension T0 of each sample under a compression area (area of the disk) of 2.0 cm$^2$ and a load of 0.49 hPa.

Next, the measurement sample was compressed to be under a load of 49.03 hPa at a pressing speed of 0.02 mm/sec, and the thickness dimension Tm under the load of 49.03 hPa was measured.

Further, the workload [N·m/m$^2$] required during compression from the thickness dimension T0 to the thickness dimension Tm was set as WC, the workload required during restoration from the thickness dimension Tm to the thickness dimension T0 was set as WC2, and the compression recovery rate RC [%] was obtained on the basis of the following formula;

$$RC\ (\%)\ =\ WC2/WC\ *\ 100$$

<Measurement of air permeability (air-permeable resistance value) >

First, each non-woven fabric was cut into a circular shape having a diameter of 100 mm and cut out to obtain a sample.

A known air permeability tester, for example, an air permeability tester manufactured by Kato-tec Corporation.: KES-F8-APL, was used to measure an air-permeable resistance value of the samples at a normal air permeability rate of 2 cm/s.

This measurement was performed five times, and the average value thereof was used as the air-permeable resistance value of each non-woven fabric.

It can be said that the higher the air-permeable resistance value, the worse the air permeability.

<Measurement result>

As indicated in Table 1, when comparing Example 1, which is a flexible non-woven fabric formed of polyethylene fibers having a fiber diameter of 14.83 $\mu$m, with Comparative Example 1, which is a non-woven fabric formed of polypropylene fibers having a fiber diameter of 12.19 $\mu$m, the MIU of Example 1 was 0.335, whereas the MIU of Comparative Example 1 was 0.238, and MMD/MIU*100 (%) of the variation coefficient to the friction coefficient of Example 1 was 2.57%, whereas MMD/MIU*100 (%) of the variation coefficient to the friction coefficient of Comparative Example 1 was 3.28%.

From this, it can be said that since the flexible non-woven fabric is formed of polyethylene fibers, which is relatively flexible, the friction coefficient MIU is relatively high, and as a result, MMD/MIU*100 (%) of the variation coefficient to the friction coefficient is low, and the variation of the friction coefficient is less and the surface of the sheet is smooth.

Accordingly, when the wearer's caretaker moves the fingertip in the plane direction with respect to the surface of the flexible non-woven fabric, the amount of fibers in contact with the fingerprint so as to stick to the fingerprint is relatively large, and a smooth tactile feel can be provided.

In addition, in comparison with Example 1 and Comparative Example 1 in which the mass of the non-woven fabric is substantially the same, the air-permeable resistance value of Example 1 is 0.0324, whereas the air-permeable resistance value of Comparative Example 1 is 0.0356, and it can be said that the flexible non-woven fabric of Example 1 is superior in the air permeability to the non-woven fabric of Comparative Example 1.

The reason for this is considered to be due to the fact that the fiber diameter of the polyethylene fiber forming the flexible non-woven fabric of Example 1 was larger than the fiber diameter of the polypropylene fiber forming the non-woven fabric of Comparative Example 1, therefore a large number of gaps were formed between the fibers and the air permeability was improved.

In addition, when comparing Examples 1 to 5 with Comparative Examples 2 and 3, MMD/MIU*100 (%) of the variation coefficient to the friction coefficient of Examples 1 to 5 was less than 6%, whereas the MMD/MIU*100 (%) of the variation coefficient to the friction coefficient of Comparative Examples 2 and 3 was more than 6%, and the distribution of the friction coefficient of the non-woven fabric formed of composite fibers was uneven compared with the distribution of the flexible non-woven fabric formed of single fibers of polyethylene fibers, which causes deterioration of the smoothness of the sheet surface.

All feature configurations that one of ordinary skill in the art can understand from the description of the present specification, the drawings, and the claims may be independently or optionally combined with one or more other feature configurations, even though described in combination only with respect to certain other features.

[Reference Signs List]

10 disposable diaper (absorbent article)
17 inner layer sheet (flexible non-woven fabric)
18 outer layer sheet (flexible non-woven fabric)
21 inner layer sheet (flexible non-woven fabric)
22 outer layer sheet (flexible non-woven fabric)
31 body side liner (flexible non-woven fabrics)
40 covering sheet (flexible non-woven fabric)

**Claims**

1. An absorbent article having an inner surface side sheet located on a skin facing surface side, an outer surface side sheet located on a non-skin facing surface side and an absorbent body interposed between the inner and outer sheets;

   wherein at least one of the inner and outer sheets is formed of a flexible non-woven fabric;
   the flexible non-woven fabric is a spun-melt nonwoven fabric;
   the flexible non-woven fabric is mainly formed of a polyethylene fiber being 100% by mass with respect to the entire flexible non-woven fabric and having a fiber diameter of 14 to 22 $\mu$m;
   MMD/MIU*100% obtained by a KES method is 2% to 6% measured as described in the description, where MMD is average deviation of friction coefficient and MIU is average friction coefficient;
   wherein a flexural rigidity value B of the flexible non-woven fabric obtained by the KES method is 0.003 x 10$^{-4}$ to 0.01 x 10$^{-4}$N·m$^2$/m measured as described in the description; and
   the range of predetermined values of MMD / MIU *100% and the flexural rigidity value B is satisfied in both the machine direction(MD) and the cross direction(CD) at the time of manufacturing the flexible non-woven fabric.

2. The absorbent article according to Claim 1,
   wherein a compression resilience rate RC by the KES method is 15% to 50% measured as described in the description.

3. The absorbent article according to Claim 1 or 2,
   wherein the flexible non-woven fabric has a mass of 10 to 30 g/m$^2$ measured as described in the description and an apparent density of 0.12 to 0.2 g/cm$^3$ under a 49.03 hPa load measured as described in the description.

4. The absorbent article according to any one of Claims 1 to 3,
   wherein the average friction coefficient MIU of the flexible non-woven fabric is at least 0.25.

**5.** The absorbent article according to any one of Claims 1 to 4,
wherein constituent fibers of the flexible non-woven fabric are bonded to each other through a plurality of fused portions, and a total area ratio of the fused portions to an area of the flexible non-woven fabric is 5% to 25%.

**6.** The absorbent article according to any one of Claims 1 to 5, wherein a specific volume under a load of 49.03 hPa is 5.0 to 10 cm$^3$/g measured as described in the description.

**Patentansprüche**

**1.** Absorptionsfähiger Artikel, der eine innere Oberflächenseitenlage, die sich auf einer der Haut zugewandten Oberflächenseite befindet, eine äußere Oberflächenseitenlage, die sich auf einer nicht der Haut zugewandten Oberflächenseite befindet, und einen absorptionsfähigen Körper, der zwischen der inneren und der äußeren Lage eingeschoben ist, aufweist;

    wobei mindestens eine der inneren und der äußeren Lage aus einem flexiblen Vliesstoff hergestellt ist;
    der flexible Vliesstoff ein Spunmelt-Vliesstoff ist;
    der flexible Vliesstoff hauptsächlich aus einer Polyethylenfaser hergestellt ist, die 100 Massen-% bezogen auf den gesamten flexiblen Vliesstoff ausmacht und einen Faserdurchmesser von 14 bis 22 μm aufweist;
    der MMD/MIU*100 %, der anhand einem KES-Verfahren erhalten wird, 2 % bis 6 %, wie in der Beschreibung beschrieben gemessen, beträgt, wobei MMD eine durchschnittliche Abweichung eines Reibungskoeffizienten ist und MIU ein durchschnittlicher Reibungskoeffizient ist;
    wobei ein Biegesteifigkeitswert B des flexiblen Vliesstoffs, der anhand dem KES-Verfahren erhalten wird, 0,003 x 10$^{-4}$ bis 0, 01 x 10$^{-4}$ N·m$^2$/m, wie in der Beschreibung beschrieben gemessen, beträgt; und
    dem Bereich von vorbestimmten Werten von MMD/MIU*100 % und der Biegesteifigkeitswert B in sowohl der Maschinenrichtung (MD) als auch der Querrichtung (CD) zur Zeit der Fertigung des flexiblen Vliesstoffs entsprochen wird.

**2.** Absorptionsfähiger Artikel nach Anspruch 1,
wobei eine Kompressionswiderstandsrate RC anhand dem KES-Verfahren 15 % bis 50 %, wie in der Beschreibung beschrieben gemessen, beträgt.

**3.** Absorptionsfähiger Artikel nach Anspruch 1 oder 2,
wobei der flexible Vliesstoff eine Masse von 10 bis 30 g/m$^2$, wie in der Beschreibung beschrieben gemessen, und eine Rohdichte von 0,12 bis 0,2 g/cm$^3$ unter einer Last von 49,03 hPa, wie in der Beschreibung beschrieben gemessen, aufweist.

**4.** Absorptionsfähiger Artikel nach einem der Ansprüche 1 bis 3,
wobei der durchschnittliche Reibungskoeffizient MIU des flexiblen Vliesstoffs mindestens 0,25 beträgt.

**5.** Absorptionsfähiger Artikel nach einem der Ansprüche 1 bis 4,
wobei einzelne Fasern des flexiblen Vliesstoffs durch eine Vielzahl von verschmolzenen Abschnitten aneinander gebondet werden und ein Gesamtoberflächenverhältnis der verschmolzenen Abschnitte zu einer Oberfläche des flexiblen Vliesstoffs 5 % bis 25 % beträgt.

**6.** Absorptionsfähiger Artikel nach einem der Ansprüche 1 bis 5, wobei ein spezifisches Volumen unter einer Last von 49,03 hPa 5,0 bis 10 cm$^3$/g, wie in der Beschreibung beschrieben gemessen, beträgt.

**Revendications**

**1.** Article absorbant comportant une feuille latérale de surface interne située sur un côté de la surface faisant face à la peau, une feuille latérale de surface externe située sur un côté de la surface ne faisant pas face à la peau et un corps absorbant interposé entre les feuilles interne et externe ;

    dans lequel au moins l'une des feuilles interne et externe est formée d'un tissu non tissé flexible ;
    le tissu non tissé flexible est un tissu non tissé filé en fusion ;
    le tissu non tissé flexible est principalement formé d'une fibre de polyéthylène représentant 100 % en masse de l'ensemble du tissu non tissé flexible et ayant un diamètre de fibre de 14 à 22 $\mu$m ;

la valeur MMD/MIU *100 % obtenue par une méthode KES, mesurée comme il est décrit dans la description, est de 2 % à 6 %, MMD étant l'écart moyen du coefficient de frottement et MIU étant le coefficient de frottement moyen ;

dans lequel une valeur de rigidité en flexion B du tissu non tissé flexible obtenue par la méthode KES, mesurée comme il est décrit dans la description, est de 0,003 x $10^{-4}$ à 0,01 x $10^{-4}$ N·m$^2$/m ; et

la plage de valeurs prédéterminées de MMD/MIU *100 % et de la valeur de rigidité en flexion B est satisfaite à la fois dans le sens machine (MD) et dans le sens travers (CD) au moment de la fabrication du tissu non tissé flexible.

2. Article absorbant selon la revendication 1,
dans lequel un taux de résistance en compression RC obtenu par la méthode KES, mesuré comme il est décrit dans la description, est de 15 % à 50 %.

3. Article absorbant selon la revendication 1 ou 2,
dans lequel le tissu non tissé flexible a une masse, mesurée comme il est décrit dans la description, de 10 à 30 g/m$^2$, et une densité apparente sous une charge de 49,03 hPa, mesurée comme il est décrit dans la description, de 0,12 à 0,2 g/cm$^3$.

4. Article absorbant selon l'une quelconque des revendications 1 à 3,
dans lequel le coefficient de frottement moyen MIU du tissu non tissé flexible est d'au moins 0,25.

5. Article absorbant selon l'une quelconque des revendications 1 à 4,
dans lequel les fibres constitutives du tissu non tissé flexible sont liées les unes aux autres par une pluralité de parties fusionnées, et un rapport de la surface totale des parties fusionnées à une surface du tissu non tissé flexible est de 5 % à 25 %.

6. Article absorbant selon l'une quelconque des revendications 1 à 5, dans lequel un volume spécifique sous une charge de 49,03 hPa, mesuré comme il est décrit dans la description, est de 5,0 à 10 cm$^3$/g.

# FIG.1

# FIG.2

# FIG.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11293554 A
- EP 3275413 A1
- EP 3117811 A1
- WO 2014050544 A1
- WO 2015182441 A1
- CA 2733026 A1
- US 2016287450 A1
- KR 20120028551 A

**Non-patent literature cited in the description**

- Standardization and Analysis of Texture Hand Evaluation. Texture Machinery Society of Japan, 10 July 1980